# EUROPÄISCHE PATENTSCHRIFT

(11) **EP 2 258 439 B1**
(45) Veröffentlichungstag und Bekanntmachung des Hinweises auf die Patenterteilung: **29.04.2020**
(21) Anmeldenummer: 10157039.8
(22) Anmeldetag: 19.03.2010
(51) Int. Cl.: A61M 25/10

(54) **Strukturierter Wirkstoff-freisetzender Ballonkatheter**
Structured drug-eluting balloon catheter
Cathéter à ballonnet avec structure à élution de médicament

(30) Priorität: 04.06.2009 US 183991 P
(43) Veröffentlichungstag der Anmeldung: 08.12.2010
(73) Patentinhaber: BIOTRONIK AG, 8180 Bülach (CH)
(72) Erfinder: Adden, Nina, 90427, Nürnberg (DE); Surber, Bettina, 8542 Gachnang (CH)
(74) Vertreter: Keck, Hans-Georg

(56) Entgegenhaltungen:
- EP-A1- 1 604 704
- WO-A2-02/43796
- US-A1- 2004 064 093
- US-A1- 2009 112 239

## Beschreibung

### Technisches Gebiet

Die vorliegende Erfindung betrifft das Gebiet der Wirkstoff-freisetzenden Ballonkatheter, sowie Verfahren zu Herstellung solcher Ballonkatheter.

### Technologischer Hintergrund und Stand der Technik

Die Angioplastie, auch perkutane transluminale Angioplastie (PTA) oder perkutane transluminale Coronarangioplastie (PTCA), ist ein Verfahren zur Erweiterung oder Wiedereröffnung von verengten oder verschlossenen Blutgefäßen (meistens Arterien, seltener auch Venen). Ein gebräuchliches Verfahren der Angioplastie ist die Ballondilatation.

Unter der Ballondilatation im Rahmen einer Angioplastie versteht man in der interventionellen Radiologie, der Kardiologie und der Angiologie eine Methode zur Aufdehnung krankhaft verengter Blutgefäße mittels eines Ballonkatheters, einem Gefäßkatheter mit daran angebrachtem Ballon, der sich erst an der verengten Stelle langsam unter hohem Druck (6-20 bar) entfaltet. Dadurch werden die Engstellen, die v.a. durch atherosklerotische Veränderungen (Gefäßverkalkung) entstehen, so gedehnt, dass sie den Blutstrom nicht mehr oder weniger stark behindern.

Dabei werden die Ballonkatheter fast immer von der Leiste aus über einen Führungsdraht und Führungskatheter in die Stenose (Engstelle) platziert und mit Druck aufgeblasen. Hierdurch wird meist die Engstelle beseitigt und eine Operation vermieden. In WO 02/43796 A2 werden Ballonkatheter mit Mikronadeln beschrieben.

Moderne Methoden im Bereich Kunststoffverarbeitung ermöglichen die Konstruktion und Weiterentwicklung solcher Ballons, um die Qualität individuell auf die Bedürfnisse der Patienten anzupassen. Wichtig sind hierbei die Flexibilität der Ballons sowie ihre Druckfestigkeit.

Der Wirkstoff-freisetzende Ballonkatheter (engl. *drug-eluting balloon* oder *drug-coated balloon*) ist eine Weiterentwicklung der herkömmlichen Ballonkatheter.

Dabei ist die Ballonoberfläche mit einem Wirkstoff oder Medikament beschichtet, das an der Stelle der Gefäßverengung durch die Dilatation des Ballons appliziert wird, um die Gefäßaufweitung pharmakologisch zu unterstützen und zu stabilisieren. Im Gegensatz zur Stent-Therapie verbleibt nach dem Eingriff kein mechanisch wirkender Fremdkörper im Körper.

Als Wirkstoff wird hierbei häufig ein Zytostatikum, wegen seines schnellen Eindringens in die Gefäßwand beispielsweise Paclitaxel verwendet. Zudem wird dem Medikament häufig ein Additiv zugesetzt, das die Aufnahme des Medikamentes in die Gefäßwand fördert.

Es sind verschiedene Methoden bekannt Wirkstoffe oder Medikamente auf einen Ballonkatheter zu applizieren:
1) Einbettung des Wirkstoffs in eine mikroporöse Oberfläche des Ballons;
2) Beschichtung des Ballons mit Wirkstoff-haltigen Polymerschichten (Basecoating, Drugdepot).

Bei den herkömmlichen Verfahren wird der größte Teil des Wirkstoffs unter die Falten des nicht dilatierten Ballons aufgebracht. Dadurch ergibt sich eine ungleichmäßige Verteilung des Wirkstoffs über die dilatierte Ballonoberfläche und später auch über die Oberfläche des dilatierten Gefäßes.

Häufig wird der Wirkstoff durch Tauchen oder Sprühen auf den Ballon aufgebracht. Beim späteren Dilatieren kann der äußerlich verbliebene Anteil des Wirkstoffs unkontrolliert abplatzen, so dass die Freisetzung des eingebrachten Wirkstoffs nicht exakt kontrolliert werden kann.

Ferner ist bekannt, daß Wirkstoff-freisetzende Ballons bis zu 80% ihrer Wirkstoffbeladung in den Blutstrom abgeben und dadurch in unerwünschter Weise die systemische Wirkstoffkonzentration erhöhen. Insbesondere bei Ballons, die in großen peripheren Blutgefäßen eingesetzt werden, können dabei Wirkstoffspiegel im Blut erreicht werden, die einer chemotherapeutischen Behandlung vergleichbar sind.

Aufgabe der vorliegenden Erfindung ist es einen oder mehrere Nachteile des Standes der Technik zu vermindern oder zu vermeiden.

### Erfindungsgemäße Lösung

Die Aufgabe wird gelöst durch Bereitstellung eines Ballonkatheters, der, mindestens auf Teilen der nach außen gewandten Oberfläche des dilatierbaren Bereichs, haarähnliche Fortsätze aufweist, wobei der durchschnittliche Durchmesser *D,* die durchschnittliche Länge *L* und der gemittelte Zentrum-zu-Zentrum-Abstand *P* der haarähnlichen Fortsätze zueinander derart ausgewählt sind, dass ein Wirkstoff auf der mit haarähnlichen Fortsätzen bedeckten Ballonoberfläche im Wesentlichen mittels Kapillarkräften fixierbar ist, wobei D aus einem Bereich von 0,1 bis 5 µm, L aus einem Bereich von 0,1 bis 5 µm und P aus einem Bereich von 0,1 bis 7 µm ausgewählt ist, und wobei das Verhältnis D zu L ausgewählt ist aus einem Bereich von 0,5 bis 2 und das Verhältnis P zu D ausgewählt ist aus einem Bereich von 1 bis 50.

Angelehnt an die Oberflächenstruktur der Innenfläche von Geckofüßen, weist eine Oberfläche des dilatierbaren Ballons eine Vielzahl von haarähnlichen Fortsätzen auf. Der Gecko kann durch eine Vielzahl an sub-mikrometer großen Härchen an seinen Füßen auch glatte Flächen senkrecht oder auch überkopf hoch laufen. Dieser Effekt ist eine Kombination von Kapillarkräften und van der Waals-Kräften. Bei dem erfindungsgemäßen Ballonkatheter werden insbesondere die Kapillarkräfte einer solchen Oberflächenstruktur genutzt, um einen Wirkstoff auf der Ballonoberfläche zu fixieren und bei Bedarf wieder abzugeben. Diese Oberflächenstrukturierung erlaubt eine gleichmäßige Beladung des Ballons mit einem Wirkstoff über eine definierte Oberfläche. Beispielsweise durch Tauchen nimmt der Ballon durch die Kapillarkräfte der Oberflächenstruktur des erfindungsgemäßen Ballonkatheters eine bestimmte Menge an Wirkstoff auf und hält diese auch nach einem Trocknungsschritt auf der Oberfläche fixiert. Es ist kein aufwendiges Applikationsverfahren notwendig und ein Abplatzen des getrockneten Wirkstoffs von der Ballonoberfläche wird vermieden.

Grundsätzlich kann für den erfindungsgemäßen Ballonkatheter jedes bekannte Ballonkathetersystem verwendet werden. Insbesondere betrifft die Erfindung einen Ballonkatheter mit einem Innenschaft, an dem ein distales Ende eines expandierbaren Ballons befestigt ist, der in einem nicht-expandierten, deflatierten Zustand an einer Aussenoberfläche des Innenschaftes zumindest teilweise anliegt. Die Erfindung betrifft insbesondere solche Katheter, die auf der Aussenseite des deflatierten Ballons einen Wirkstoff tragen sollen, der nach Einführen in ein Gefäß und Dilatation einer Gefäßverengung durch Inflation des Ballons mit einem Fluid am vorgesehenen Ort an eine Gefäßwand gedrückt wird und mindestens teilweise dort abgegeben wird.

Ballonkatheter der vorgesehenen Art weisen üblicherweise neben einem Innenschaft und dem Ballon auch einen Außenschaft auf, der wenigstens bis zu einem proximalen Ende des Ballons reicht und mit diesem fluiddicht verbunden ist. Zwischen Innen- und Außenschaft des Katheters ist üblicherweise eine in Längsrichtung des Katheters von seinem proximalen Ende bis ins Innere des Ballons reichende Fluidleitung vorgesehen, die sich beispielsweise daraus ergibt, dass der Außenschaft einen Innendurchmesser besitzt, der größer ist, als ein Außendurchmesser des Innenschaftes.

Im Inneren des Innenschaftes ist ein vom Innenschaft eingeschlossener, sich in Längsrichtung des Innenschaftes erstreckender Hohlraum als Lumen vorgesehen. Dieses Lumen dient beispielsweise der Aufnahme eines Mandrins oder eines Führungsdrahtes. Katheter und Führungsdraht sind dann beispielsweise so ausgebildet, dass der Führungsdraht an der distalen Spitze des Katheters austreten kann und vom proximalen Ende her zu steuern ist. Der Führungsdraht wird zum Beispiel mit Hilfe von Steuermitteln so ausgelenkt, dass er auch in abzweigende Blutgefässe leicht einzuführen ist. Der Ballonkatheter kann dann entlang des Führungsdrahtes nachgeschoben werden.

Unabhängig von der Art des Katheters, insbesondere hinsichtlich der Gestaltung der Führungsmittel, weisen Ballonkatheter an ihrem distalen Ende den bereits erwähnten, expandierbaren Ballon auf. Während des Einführens des Ballonkatheters ist der Ballon komprimiert und liegt eng am Innenschaft des Katheters an. Durch Inflatieren des Ballons mit einem Fluid kann dieser expandiert werden. Dieses Expandieren des Ballons geschieht, sobald der Ballon bis zu der bestimmungsgemäßen Position geführt ist. Durch das Expandieren des Ballons wird eine Oberfläche des Ballons an eine Gefäßwand anlegt. Dies geschieht beispielsweise zu dem Zweck, Gefäßverengungen (Stenosen) mittels des Ballonkatheters zu weiten.

Der erfindungsgemäße Ballonkatheter weist mindestens auf Teilen der nach außen gewandten Oberfläche des dilatierbaren Bereichs haarähnliche Fortsätze auf. Insbesondere können solche Bereiche des Ballons oder Teile davon mit haarähnlichen Fortsätzen versehen sein, die nach Inflatieren des Ballons in Kontakt mit einer Gefäßwand gelangen. Bevorzugt weisen mindestens 20% der Ballonoberfläche haarähnliche Fortsätze auf, besonders bevorzugt weisen 30% bis 100% der Ballonoberfläche haarähnliche Fortsätze auf. Die haarähnlichen Fortsätze weisen eine Form auf, die auf der Ballonoberfläche fußt und sich darüber erhebt. Insbesondere können die haarähnlichen Fortsätze eine oder mehrere Seitenflächen aufweisen und ggf. eine Oberfläche, die von der Ballonoberfläche abgewandt ist und bei Inflatieren des Ballons in Kontakt mit einer Gefäßwand gebracht werden kann. Bevorzugt sind die haarähnlichen Fortsätze im Wesentlichen zylinderförmig.

Die haarähnlichen Fortsätze können grundsätzlich aus jedem Material gefertigt werden, dass sich mit einer Ballonkatheteroberfläche verbinden lässt und das ausreichend verträglich ist für einen Einsatz innerhalb eines menschlichen Gefäßes. Die haarähnlichen Strukturen bestehen bevorzugt aus Materialien oder Materialzusammensetzungen, die eine Festigkeit und Steifigkeit aufweisen, so dass die haarähnlichen Fortsätze aufrecht von der Ballonoberfläche abstehen können und nicht in sich selbst kollabieren oder abknicken. Die haarähnlichen Fortsätze können insbesondere aus demselben Material gefertigt sein, aus dem die Außenoberfläche des Ballons des Katheters gefertigt ist. Geeignete Materialien (z.B. Polymere) sind dem Fachmann bekannt.

Eine Oberfläche mit haarähnlichen Fortsätzen kann mit unterschiedlichen Verfahren hergestellt werden. Beispielsweise können durch lithographische Verfahren, wie z.B. Elektronenstrahllithographie und Laserlithographie, oder durch Ätzverfahren negative Formen erzeugt werden. In einem anschließenden Gussverfahren wird dann ausgehend von der negativen Form die positive Oberfläche mit haarähnlichen Fortsätzen erzeugt (siehe beispielsweise A.K. Geim et al., Nature Mater. 2, 461-463 (2003) und H. Lee, B.P. Lee and P.B. Messersmith, Nature 448, 338-341 (2007)).

Der durchschnittliche Durchmesser D der haarähnlichen Fortsätze, die durchschnittliche Länge *L* der haarähnlichen Fortsätze, sowie der gemittelte Zentrum-zu-Zentrum Abstand *P* der haarähnlichen Fortsätze zueinander sind Parameter, die die Größe der erzielbaren Kapillarkräfte beeinflussen. *D* kann bestimmt werden, in dem für eine ausgewählte Fläche die Durchmesser aller haarähnlichen Fortsätze bestimmt, addiert und die erhaltene Summe durch die Anzahl der vermessenen haarähnlichen Fortsätze geteilt wird. *L* kann bestimmt werden, in dem für eine ausgewählte Fläche die Länge aller haarähnlichen Fortsätze bestimmt, addiert und die erhaltene Summe durch die Anzahl der vermessenen haarähnlichen Fortsätze geteilt wird. *P* kann bestimmt werden, in dem für eine ausgewählte Fläche die Abstände aller haarähnlichen Fortsätze zum jeweils nächstgelegenen haarähnlichen Fortsatz bestimmt werden, wobei ausgehend von einem Zentrum eines ersten haarähnlichen Fortsatzes zum Zentrum des nächstgelegenen haarähnlichen Fortsatzes gemessen wird. Die erhaltenen Abstandswerte werden addiert und die erhaltene Summe wird durch die Anzahl der vermessenen haarähnlichen Fortsätze geteilt.

Erfindungsgemäß sind *D, L* und *P* derart ausgewählt, dass ein gegebener Wirkstoff auf der mit haarähnlichen Fortsätzen bedeckten Ballonoberfläche fixierbar ist. Auch die Materialeigenschaften des Materials das zur Herstellung der haarähnlichen Fortsätze eingesetzt wurde, beeinflusst die erzielbaren Kapillarkräfte, beispielsweise die Hydrophobizität der Materialoberfläche. Die benötigten Kapillarkräfte um einen bestimmten Wirkstoff oder eine Lösung enthaltend diesen Wirkstoff erfindungsgemäß auf der Ballonkatheteroberfläche zu fixieren, sind nicht für jeden Wirkstoff oder für jede Lösung gleich und müssen daher für jede Kombination von Wirkstoff und erfindungsgemäßem Ballonkatheter neu bestimmt werden. Der Fachmann kann ohne Schwierigkeiten durch Routineversuche eine Kombination aus *D, L* und *P* ermitteln, die geeignet ist einen gegebenen Wirkstoff in gewünschtem Maße auf der mit haarähnlichen Fortsätzen bedeckten Ballonoberfläche zu fixieren. Dazu können Testoberflächen bereitgestellt werden, die haarähnliche Fortsätze mit verschiedenen Kombinationen von *D, L* und *P* aufweisen. Diese Testoberflächen können dann mit dem gewünschten Wirkstoff oder der gewünschten Wirkstofflösung in Kontakt gebracht werden, beispielsweise durch Tauchen oder Besprühen. Ggf. kann sich ein Trocknungsschritt anschließen, bei dem ggf. Lösungsmittel entfernt werden können. Anschließend wir die Menge an Wirkstoff bestimmt, die auf der Testoberfläche fixiert wurde.

Erfindungsgemäß wird D aus einem Bereich von 0,1 bis 5 µm, bevorzugt von 0,2 bis 1 µm ausgewählt. *L* ist ausgewählt aus einem Bereich von 0,1 bis 5 µm, bevorzugt von 0,15 bis 2 µm. *P* ist ausgewählt aus einem Bereich von 0,1 bis 7 µm, bevorzugt von 0,5 bis 3 µm. D und L sind derart ausgewählt, dass sich ein Verhältnis von *D* zu *L* ergibt, welches ausgewählt ist aus einem Bereich von 0,5 bis 2, bevorzugt 0,75 bis 1,5. P und D sind derart ausgewählt, dass sich ein Verhältnis von *P* zu *D* ergibt, das ausgewählt ist aus einem Bereich von 1 bis 50, bevorzugt von 1,5 bis 10.

Der erfindungsgemäße Ballonkatheter kann haarähnliche Fortsätze aufweisen, wobei die haarähnlichen Fortsätze beschichtet sind mit einem Polymer mit Catechol-Seitengruppen. Geeignete Polymere sowie Polymerzusammensetzungen und Verfahren zur Beschichtung sind in WO 08/091386 offenbart. Solche Polymere können Katecholamine aufweisen, wie Dopamin oder die Aminosäure 3,4-dihydroxy-L-Phenylalanin, auch als DOPA bekannt. Ein besonders bevorzugtes Polymer ist poly(Dopamin Methacrylamid (DMA) - co - Methoxyethyl Acrylat (MEA)). Bevorzugte Polymere weisen Catechol-Seitengruppen auf, wobei die Catechol-Seitengruppen mindestens 5 Gew.% des Gesamtpolymergewichts ausmachen, besonders bevorzugt von 10 Gew.% bis 70 Gew.%. Die Beschichtung der haarähnlichen Fortsätze mit einem Polymer mit Catechol-Seitengruppen kann eine Schichtdicke aufweisen von weniger als 100 nm, bevorzugt ist die Schichtdicke ausgewählt aus einem Bereich von 1 nm bis 50 nm.

Die vorliegende Erfindung bezieht sich auch auf einen Ballonkatheter, bei dem ein Wirkstoff oder eine Lösung enthaltend einen Wirkstoff mindestens auf Teilen der Oberfläche der haarähnlichen Fortsätze fixiert ist. Dabei kann der Ballonkatheter einen einzigen oder mehrere verschiedene Wirkstoffe aufweisen, die als Mischung oder in verschiedenen Bereichen des Ballonkatheters getrennt fixiert vorliegen können. Bevorzugt weist der Ballonkatheter mindestens einen Wirkstoff auf, der ein Zytostatikum, insbesondere Paclitaxel oder Docetaxel ist.

Zusätzlich zu dem Wirkstoff oder den Wirkstoffen können auf Teilen der Oberfläche der haarähnlichen Fortsätze ein oder mehrere Additive fixiert sein. Als Additive können Substanzen verwendet werden, die als Additiv für Medizinprodukte zugelassen sind, wie beispielsweise Antioxidantien, Weichmacher und/oder Kontrastmittel. Additive können die Aufnahme eines Wirkstoffs in die Zellen fördern. Geeignete Antioxidantien sind beispielsweise Ascorbylpalmitat (E304), Butylhydroxyanisol (E320), Butylhydroxytoluol (E321), Gallat, Propylgallat (E310), Octylgallat (E311), Dodecylgallat (Laurylgallat, E312), Lecithine (E322), Tocopherol (Vitamin E, E306), α-Tocopherol (E307), γ-Tocopherol (E308), δ-Tocopherol (E309), Tocotrienol (Vitamin E, E306). Geeignete Kontrastmittel umfassen Iopamidol (Isovue 370), Iohexanol (Omnipaque 350), Ioxilan (Oxilan), Iopromide, Iodixanol (Visipaque 320).

Die vorliegende Erfindung bezieht sich auch auf ein Verfahren zur Herstellung eines Wirkstoff-freisetzenden Ballonkatheters, wobei die Oberfläche eines erfindungsgemäßen Ballonkatheters mindestens in dem Bereich in dem der Ballonkatheter haarähnliche Fortsätze aufweist mindestens Teilweise mit einer Lösung enthaltend einen Wirkstoff oder ein Wirkstoffgemisch in Kontakt gebracht wird und ggf. anschließend einem Trocknungsschritt unterzogen wird. Bevorzugt wird der Wirkstoff oder die Wirkstofflösung durch Tauchen, Bedampfen, Besprühen oder Beschichten mit den haarähnlichen Fortsätzen des Ballonkatheters in Kontakt gebracht. Geeignete Verfahren sind dem Fachmann bekannt. Die durch die erfindungsgemäße Anordnung der haarähnlichen Fortsätze hervorgerufenen Kapillarkräfte sorgen dafür, dass der Wirkstoff oder die Wirkstofflösung auf der Oberfläche des Ballonkatheters fixiert wird.

Die Erfindung bezieht sich auch auf einen Ballonkatheter der mit einem erfindungsgemäßen Verfahren herstellbar ist.

Die Erfindung schließt auch erfindungsgemäße Ballonkatheter ein, zur Verwendung in der Angioplastie, insbesondere der perkutanen transluminalen Angioplastie oder der perkutanen transluminalen Coronarangioplastie.

### Figuren:

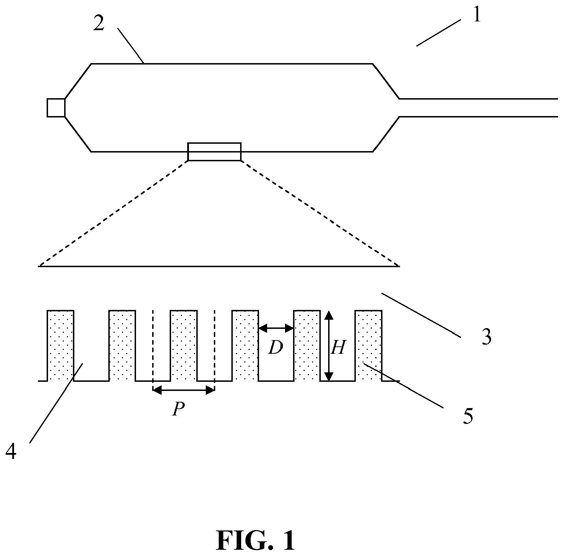

- FIG. 1: zeigt eine beispielhafte Ausführungsform eines erfindungsgemäßen Ballonkatheters.

Die Erfindung wird nachfolgend anhand eines Ausführungsbeispiels näher erläutert.

Der in FIG. 1 gezeigte Ballonkatheter 1 ist eine beispielhafte Ausführungsform der erfindungsgemäßen Ballonkatheter. Der Ballonkatheter 1 weist einen dilatierbaren Bereich 2 auf. Mindestens auf einem Teil der nach außen gewandten Oberfläche 3 des dilatierbaren Bereichs 2 befinden sich eine Mehrzahl haarähnlicher Fortsätze 4 mit einem bestimmten durchschnittlichen Durchmesser *D,* einer bestimmten durchschnittlichen Länge *L* und einem bestimmten gemittelten Zentrum-zu-Zentrum Abstand *P.* Dabei sind *D, L* und *P* so ausgewählt, dass Kapillarkräfte zwischen den einzelnen haarähnlichen Fortsätzen 4 eine Wirkstofflösung 5 mindestens auf Teilen der Oberfläche der haarähnlichen Fortsätze 4 fixieren.

### Bezugszeichenliste:

- 1: Ballonkatheter
- 2: dilatierbarer Bereich
- 3: Teil der nach außen gewandten Oberfläche des dilatierbaren Bereichs 2
- 4: haarähnlicher Fortsatz
- 5: Wirkstofflösung

## Patentansprüche

1. Ballonkatheter (1), **dadurch gekennzeichnet, dass** der Ballonkatheter mindestens auf Teilen der nach außen gewandten Oberfläche (3) des dilatierbaren Bereichs (2) haarähnliche Fortsätze (4) aufweist, wobei der durchschnittliche Durchmesser *D,* die durchschnittliche Länge *L* und der gemittelte Zentrum-zu-Zentrum-Abstand *P* der haarähnlichen Fortsätze (4) zueinander derart ausgewählt sind, dass ein Wirkstoff auf der mit haarähnlichen Fortsätzen (4) bedeckten Ballonoberfläche im Wesentlichen mittels Kapillarkräften fixierbar ist, wobei *D* aus einem Bereich von 0,1 bis 5 µm, *L* aus einem Bereich von 0,1 bis 5 µm und *P* aus einem Bereich von 0,1 bis 7 µm ausgewählt ist, und wobei das Verhältnis *D* zu *L* ausgewählt ist aus einem Bereich von 0,5 bis 2 und das Verhältnis *P* zu *D* ausgewählt ist aus einem Bereich von 1 bis 50.

2. Ballonkatheter (1) nach Anspruch 1, wobei die haarähnlichen Fortsätze (4) im Wesentlichen zylinderförmig sind.

3. Ballonkatheter (1) nach einem der vorhergehenden Ansprüche, wobei *D* ausgewählt ist aus einem Bereich von 0,2 bis 1 µm.

4. Ballonkatheter (1) nach einem der vorhergehenden Ansprüche, wobei *L* ausgewählt ist aus einem Bereich von 0,15 bis 2 µm.

5. Ballonkatheter (1) nach einem der vorhergehenden Ansprüche, wobei *P* ausgewählt ist aus einem Bereich von 0,5 bis 3 µm.

6. Ballonkatheter (1) nach einem der vorhergehenden Ansprüche, wobei das Verhältnis *D* zu *L* ausgewählt ist aus einem Bereich 0,75 bis 1,5.

7. Ballonkatheter (1) nach einem der vorhergehenden Ansprüche, wobei das Verhältnis *P* zu *D* ausgewählt ist aus einem Bereich von 1,5 bis 10.

8. Ballonkatheter (1) nach einem der vorhergehenden Ansprüche, wobei die haarähnlichen Fortsätze (4) beschichtet sind mit einem Polymer mit Catechol-Seitengruppen.

9. Ballonkatheter (1) nach Anspruch 8, wobei die Beschichtung eine Schichtdicke aufweist von weniger als 100 nm, bevorzugt ist die Schichtdicke ausgewählt aus einem Bereich von 5 nm bis 50 nm.

10. Ballonkatheter (1) nach einem der vorhergehenden Ansprüche, wobei ein Wirkstoff oder eine Lösung enthaltend einen Wirkstoff (5) mindestens auf Teilen der Oberfläche der haarähnlichen Fortsätze (4) fixiert ist.

11. Ballonkatheter (1) nach Anspruch 10, wobei der Wirkstoff ein Zytostatikum ist, bevorzugt Paclitaxel oder Docetaxel.

12. Verfahren zur Herstellung eines Wirkstoff-freisetzenden Ballonkatheters (1), wobei die Oberfläche eines Ballonkatheters (1) nach einem der Ansprüche 1 bis 8 in dem Bereich in dem der Ballonkatheter haarähnliche Fortsätze (4) aufweist mindestens Teilweise mit einer Lösung enthaltend einen Wirkstoff oder ein Wirkstoffgemisch (5) in Kontakt gebracht wird, bevorzugt durch tauchen, bedampfen, besprühen oder beschichten.

## Claims

1. A balloon catheter (1), **characterised in that** the balloon catheter, at least on parts of the outwardly turned surface (3) of the dilatable region (2), has hair-like extensions (4), wherein the average diameter *D,* the average length *L,* and the average centre-to-centre distance *P* of the hair-like extensions (4) to each other is selected such that a drug on the balloon surface covered with hair-like extensions (4) is fixable substantially by means of capillary forces, wherein *D* is selected from a range of from 0.1 to 5 µm, *L* is selected from a range of from 0.1 to 5 µm, and *P* is selected from a range of from 0.1 to 7 µm, and wherein the ratio *D* to *L* is selected from a range of from 0.5 to 2 and the ratio *P* to *D* is selected from a range of from 1 to 50.

2. The balloon catheter (1) according to claim 1, wherein the hair-like extensions (4) are substantially cylindrical.

3. The balloon catheter (1) according to either one of the preceding claims, wherein *D* is selected from a range of from 0.2 to 1 µm.

4. The balloon catheter (1) according to any one of the preceding claims, wherein *L* is selected from a range of from 0.15 to 2 µm.

5. The balloon catheter (1) according to any one of the preceding claims, wherein *P* is selected from a range of from 0.5 to 3 µm.

6. The balloon catheter (1) according to any one of the preceding claims, wherein the ratio *D* to *L* is selected from a range of from 0.75 to 1.5.

7. The balloon catheter (1) according to any one of the preceding claims, wherein the ratio *P* to *D* is selected from a range of from 1.5 to 10.

8. The balloon catheter (1) according to any one of the preceding claims, wherein the hair-like extensions (4) are coated with a polymer having catechol side groups.

9. The balloon catheter (1) according to claim 8, wherein the coating has a layer thickness of less than 100 nm, and the layer thickness is preferably selected from a range of from 5 nm to 50 nm.

10. The balloon catheter (1) according to any one of the preceding claims, wherein a drug or a solution containing a drug (5) is fixed at least on parts of the surface of the hair-like extensions (4).

11. The balloon catheter (1) according to claim 10, wherein the drug is a cytostatic agent, preferably Paclitaxel or Docetaxel.

12. A method for producing a drug-eluting balloon catheter (1), wherein the surface of a balloon catheter (1) according to any one of claims 1 to 8 is brought into contact, at least in part, with a solution containing a drug or a drug mixture (5) in the region in which the balloon catheter has hair-like extensions (4), preferably by dipping, steam treatment, spraying or coating.

## Revendications

1. Cathéter à ballonnet (1), **caractérisé en ce que** le cathéter à ballonnet présente des protubérances (4) ressemblant à des cheveux sur au moins des parties de la surface (3) de la zone dilatable (2) orientée vers l'extérieur, où le diamètre moyen *D,* la longueur moyenne *L* et la distance de centre à centre moyennée *P* des protubérances (4) ressemblant à des cheveux sont choisis les uns par rapport aux autres qu'une matière active peut être fixée sur la surface du ballonnet recouverte avec les protubérances (4) ressemblant à des cheveux essentiellement au moyen de forces capillaires, où *D* est choisi dans une plage de 0,1 à 5 µm, *L* dans une plage de 0,1 à 5 µm et *P* dans une plage de 0,1 à 7 µm, et où le rapport *D* sur *L* est choisi dans une plage de 0,5 à 2 et le rapport *P* sur *D* est choisi dans une plage de 1 à 50.

2. Cathéter à ballonnet (1) selon la revendication 1, dans lequel les protubérances (4) ressemblant à des cheveux ont essentiellement une forme de cylindre.

3. Cathéter à ballonnet (1) selon l'une des revendications précédentes, dans lequel *D* est choisi dans une plage de 0,2 à 1 µm.

4. Cathéter à ballonnet (1) selon l'une des revendications précédentes, dans lequel *L* est choisi dans une plage de 0,15 à 2 µm.

5. Cathéter à ballonnet (1) selon l'une des revendications précédentes, dans lequel *P* est choisi dans une plage de 0,5 à 3 µm.

6. Cathéter à ballonnet (1) selon l'une des revendications précédentes, dans lequel le rapport *D* sur *L* est choisi dans une plage de 0,75 à 1,5.

7. Cathéter à ballonnet (1) selon l'une des revendications précédentes, dans lequel le rapport *P* sur *D* est choisi dans une plage de 1,5 à 10.

8. Cathéter à ballonnet (1) selon l'une des revendications précédentes, dans lequel les protubérances (4) ressemblant à des cheveux sont revêtues avec un polymère doté de groupes latéraux de type catéchol.

9. Cathéter à ballonnet (1) selon la revendication 8, dans lequel le revêtement présente une épaisseur de couche de moins de 100 nm, de préférence, l'épaisseur de couche est choisie dans une plage de 5 nm à 50 nm.

10. Cathéter à ballonnet (1) selon l'une des revendications précédentes, dans lequel une matière active ou une solution contenant une matière active (5) est fixée au moins sur des parties de la surface des protubérances (4) ressemblant à des cheveux.

11. Cathéter à ballonnet (1) selon la revendication 10, dans lequel la matière active est un cytostatique, de préférence, du Paclitaxel ou du Docétaxel.

12. Procédé de fabrication d'un cathéter à ballonnet (1) libérant une matière active, dans lequel la surface d'un cathéter à ballonnet (1) selon l'une des revendications 1 à 8, dans la zone dans laquelle le cathéter à ballonnet présente des protubérances (4) ressemblant à des cheveux, est mise en contact au moins partiellement avec une solution contenant une matière active ou un mélange de matières actives (5), de préférence par une immersion, une vaporisation, une pulvérisation ou un revêtement.
